# EUROPÄISCHE PATENTSCHRIFT

(11) **EP 0 507 277 B1**
(45) Veröffentlichungstag und Bekanntmachung des Hinweises auf die Patenterteilung: **08.07.1998**
(21) Anmeldenummer: 92105614.9
(22) Anmeldetag: 01.04.1992
(51) Int. Cl.: C07D 239/52, C07C 333/12, C07C 307/04, C07D 295/215

(54) **Verfahren zur Herstellung von Sulfonylharnstoffen**
Process for the preparation of sulphonylureas
Procédé pour la préparation de sulfonylurées

(30) Priorität: 02.04.1991 DE 4110636
(43) Veröffentlichungstag der Anmeldung: 07.10.1992
(73) Patentinhaber: Hoechst Schering AgrEvo GmbH, 13342 Berlin (DE)
(72) Erfinder: Schlegel, Günter, Dr., W-6237 Liederbach (DE); Mildenberger, Hilmar, Dr., W-6233 Kelkheim (Taunus) (DE)

(56) Entgegenhaltungen:
- EP-A- 131 258
- EP-A- 0 004 163
- EP-A- 0 091 593
- EP-A- 0 232 067
- EP-A- 0 234 352
- EP-A- 0 342 569
- EP-A- 0 388 994
- DE-A- 3 105 453
- DE-B- 1 164 398
- DE-B- 1 201 337
- US-A- 4 391 976
- HOUBEN-WEYL, Methoden der Organischen Chemie, Band E4, 1983, GEORG THIEME VERLAG, STUTTGART, DE

## Beschreibung

Die Erfindung betrifft Verfahren zur Herstellung von Herbiziden aus der Gruppe der heterocyclisch substituierten Sulfonylharnstoffe, speziell der Verbindungen der Formel I, 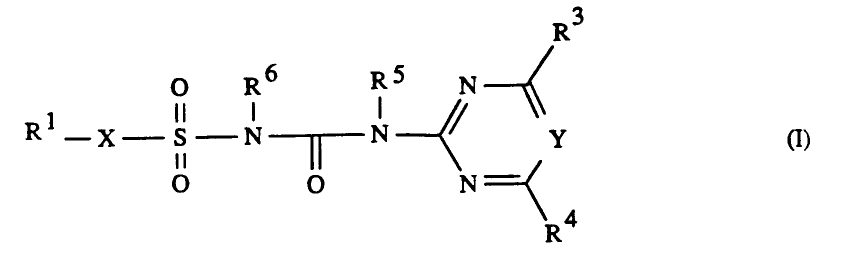 worin
- X: Sauerstoff , -O-NR²- oder -SO₂-NR²-,
- Y: ein Stickstoffatom oder CH ,
- R¹: (C₁-C₆)-Alkyl, (C₂-C₆)-Alkenyl oder (C₂-C₆)-Alkinyl, wobei jeder der genannten 3 Reste unsubstituiert oder ein- oder mehrfach durch Reste aus der Gruppe Halogen, (C₁-C₄)-Alkoxy und (C₁-C₄)-Alkoxycarbonyl substituiert ist,
oder, im Falle von X = Sauerstoff, auch Phenyl, das unsubstituiert oder durch ein oder mehrere Reste aus der Gruppe Halogen, Nitro, (C₁-C₄)-Alkyl, (C₁-C₄)-Haloalkyl, (C₁-C₄)-Alkoxy, (C₁-C₄)-Haloalkoxy oder (C₁-C₄)-Alkoxycarbonyl substituiert ist,
- R²: Wasserstoff, (C₁-C₆)-Alkyl, (C₂-C₆)-Alkenyl, (C₂-C₆)-Alkinyl oder (C₃-C₆)-Cycloalkyl,
- R³,R⁴: unabhängig voneinander Wasserstoff, (C₁-C₄)-Alkyl oder (C₁-C₄)-Alkoxy, wobei jeder der letztgenannten zwei Reste unsubstituiert oder ein- oder mehrfach durch Reste aus der Gruppe Halogen, Alkoxy und Alkylthio substituiert ist, oder Halogen, (C₁-C₄)-Alkylthio, (C₁-C₄)-Alkylamino oder Di-[(C₁-C₄)-alkyl]-amino und
- R⁵,R⁶: unabhängig voneinander Wasserstoff oder (C₁-C₄)-Alkyl
bedeuten sowie, falls einer oder jeder der Reste R⁵ und R⁶ Wasserstoff bedeutet, deren physiologisch verträglichen Salze mit Säuren oder Basen.

Verbindungen der Formel I sind bekannt und werden als Pflanzenschutzmittel mit herbizider Wirkung eingesetzt; siehe EP-A-0131258, EP-A-0342569 und EP-A-4163. Darin sind auch einige Verfahren zitiert oder beschrieben, nach denen Verbindungen der Formel I hergestellt werden können.

Nachteilig bei den bekannten Verfahren sind die relativ niedrigen Ausbeuten von höchstens etwa 65-70%. Dadurch bedingt fallen vergleichsweise große Mengen an Verunreinigungen und Nebenprodukten an, die bei Anwendung im großtechnischen Maßstab Abfall bedeuten, der aufwendig entsorgt werden muß, z. B. durch Verbrennung. Die bekannten Verfahren sind deshalb aus ökologischer als auch ökonomischer Sicht für die Durchführung im industriellen, großtechnischen Maßstab ungünstig. Außerdem tritt bei solch niedriger Ausbeute ein drastischer Verlust an eingesetzten Ausgangsmaterialien ein, was die Wirtschaftlichkeit der Verfahren schmälert.

Es wurde nun ein neues Verfahren gefunden, nach dem die Verbindungen der Formel I in überraschend hoher Ausbeute und Reinheit herstellbar sind und das sich für die Durchführung im großtechnischen Maßstab eignet.

Gegenstand der vorliegenden Erfindung ist ein Verfahren zur Herstellung der genannten Verbindungen der Formel I oder deren Salze, dadurch gekennzeichnet, daß man Verbindungen der Formel II, 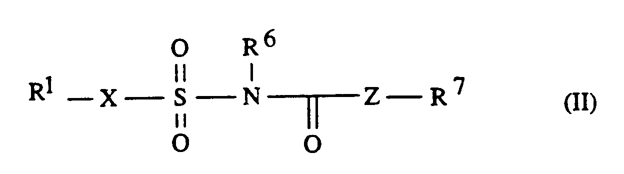 worin
- Z: ein Schwefelatom oder eine Gruppe der Formel -NR⁸- und
- R⁷,R⁸: unabhängig voneinander Wasserstoff, (C₁-C₆)-Alkyl, Phenyl oder Benzyl, wobei die genannten aromatischen Reste unsubstituiert oder durch ein oder mehrere Reste aus der Gruppe Halogen, Alkyl und Alkoxy substituiert sind, oder im Falle Z = NR⁸ die Reste R⁷ und R⁸ auch gemeinsam mit dem Stickstoffatom einen 5- bis 7-gliedrigen Ring, der ein weiteres Stickstoff- oder Sauerstoffatom enthalten und durch einen oder mehrere Substituenten aus der Gruppe Halogen und Alkyl substituiert sein kann,
bedeuten und R¹,R⁶ und X wie in Formel I definiert sind,
mit Verbindungen der Formel III, 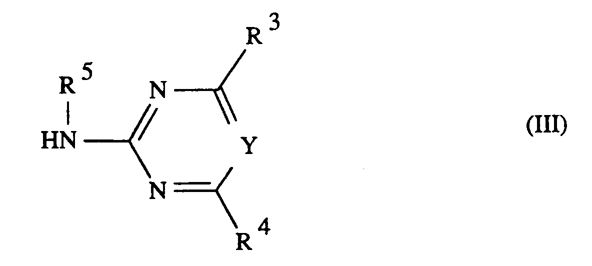 worin R³,R⁴,R⁵ und Y wie in Formel I definiert sind, umsetzt.

In den genannten Formeln bedeutet Alkyl geradkettiges oder verzweigtes Alkyl; dies gilt entsprechend für das Kohlenwasserstoffteil in den übrigen Resten, wie Alkoxy, Haloalkyl, Haloalkoxy, Alkylcarbonyl, Alkylamino, Alkenyl, Alkinyl, Alkylsulfonyl usw. Halogen bedeutet Fluor, Chlor, Brom oder Jod, vorzugsweise Fluor, Chlor oder Brom. Haloalkyl bedeutet Alkyl, das durch ein oder mehrere Halogenatome substituiert ist. Entsprechendes gilt für Haloalkoxy. Wenn nicht speziell angegeben, ist bei Alkylresten und für den Kohlenwasserstoffteil in den genannten übrigen Resten niederes Alkyl bzw. ein Rest mit 1 bis 4 C-Atomen bevorzugt.

Unter den erfindungsgemäßigen Verfahren zur Herstellung der Verbindungen der Formel I sind diejenigen von besonderem Interesse, in denen R¹X einen Rest N-(C₁-C₆)-Alkylsulfonyl-N-(C₁-C₃)-alkyl-amino oder (C₁-C₄)-Alkoxyphenoxy, R³ und R⁴ unabhängig voneinander (C₁-C₂)-Alkyl oder (C₁-C₂)-Alkoxy und R⁵ und R⁶ Wasserstoff oder Methyl bedeuten. Vorzugsweise ist dabei R¹X N-[(C₁-C₃)-Alkylsulfonyl]-N-[(C₁-C₂)-alkyl]-amino, insbesondere N-(Methylsulfonyl)-N-(methyl)-amino, N-(Methylsulfonyl)-N-(ethyl)-amino, N-(Ethylsulfonyl)-N-(methyl)-amino, N-(Ethylsulfonyl)-N-(ethyl)-amino oder N-(n-Propylsulfonyl)-N-(methyl)-amino oder (C₁-C₃)-Alkoxyphenoxy, insbesondere 2-Methoxy-phenoxy, 2-Ethoxy-phenoxy, 2-n-Propoxy-phenoxy oder 2-Isopropoxy-phenoxy, R³ und R⁴ unabhängig voneinander (C₁-C₂)-Alkyl oder (C₁-C₂)-Alkoxy, insbesondere Methyl oder Methoxy, und R⁵ und R⁶ unabhängig voneinander Wasserstoff oder Methyl.

Von besonderem Interesse sind außerdem erfindungsgemäße Verfahren, in denen
- Z: ein Schwefelatom und
- R⁷: (C₁-C₄)-Alkyl, insbesondere Methyl oder Ethyl, Phenyl oder Benzyl, wobei die genannten aromatischen Reste unsubstituiert oder durch ein oder mehrere Reste aus der Gruppe Halogen, (C₁-C₂)-Alkyl und (C₁-C₂)-Alkoxy substituiert sind,
bedeuten und R¹,R⁶ und X wie in Formel I definiert sind.

Von besonderem Interesse sind auch erfindungsgemäße Verfahren, in denen
- Z: eine Gruppe der Formel -NR⁸-,
- R⁷: Wasserstoff oder (C₁-C₄)-Alkyl, Phenyl oder Benzyl, wobei die genannten aromatischen Reste unsubstituiert oder durch ein oder mehrere Reste aus der Gruppe Halogen, (C₁-C₂)-Alkyl und (C₁-C₂)-Alkoxy substituiert sind und
- R⁸: Wasserstoff oder (C₁-C₄)-Alkyl oder
- R⁷,R⁸: gemeinsam mit dem Stickstoffatom einen 5- bis 7-gliedrigen Ring, der ein weiteres Stickstoff- oder Sauerstoffatom enthalten und durch einen oder mehrere Substituenten aus der Gruppe Halogen, (C₁-C₄)-Alkyl substituiert sein kann, beispielsweise N-Morpholinyl, N-Piperidinyl, N-Piperazinyl, N-Pyrrolidinyl, N-Imidazolinyl, N-Pyrazolidinyl oder N-Oxazolinyl,
bedeuten und R¹,R⁶ und X wie in Formel I definiert sind .

Die Ausbeuten nach dem erfindungsgemäßen Verfahren liegen vergleichsweise hoch, z. B. bei 95 % d.Th. und mehr, wobei die Reinheiten der entstehenden Sulfonylharnstoffe der Formel I oft größer als 95 Gew.-% sind.

Das erfindungsgemäße Verfahren wird in der Regel in Gegenwart eines unter den Reaktionsbedingungen inerten anorganischen oder organischen Lösungsmittels oder Gemischen zweier oder mehrerer entsprechender Lösungsmittel durchgeführt. Beispiele für geeignete Lösungsmittel sind aliphatische oder vorzugsweise aromatische, gegebenenfalls halogenierte Kohlenwasserstoffe, aprotische polare organische Lösungsmittel, wie Dialkylalkanoylamide, Dialkylsulfoxide, Polyalkylenglykoldialkylether, N-alkylierte cyclische Amide und Nitrile sowie Mischungen der genannten Lösungsmittel.

Bevorzugt sind Lösungsmittel wie z. B. Toluol, Xylol, Chlorbenzol, Dimethylformamid, Dimethylsulfoxid, Sulfolan, Di-, Tri- oder Tetraethylenglykoldialkylether, insbesondere - dimethyl oder -diethylether, N-Methylpyrrolidon, Acetonitril oder auch Mischungen aus zwei oder mehreren der genannten Lösungsmittel.

Das erfindungsgemäße Verfahren kann auch in wäßrigem, z. B. rein wäßrigem Medium durchgeführt werden.

Es ist in der Regel vorteilhaft, die Verbindung der Formel II im Verhältnis zur Verbindung der Formel III äquimolar oder in geringem Überschuß einzusetzen. Bevorzugt ist ein Molverhältnis für II:III von 1:1 bis 1,2:1, insbesondere 1:1 bis 1,1:1.

Die Reaktionstemperaturen liegen vorzugsweise bei 0°C bis 140 °C, insbesondere bei 20°C bis 132°C.

Ein Vorteil des erfindungsgemäßen Verfahrens besteht darin, daß die aus den Verbindungen der Formel II abgespaltene Verbindung der Formel IV,

R⁷―Z―H (IV)

worin R⁷ und Z wie oben definiert sind, bei der Durchführung des erfindungsgemäßen Verfahrens wieder quantitativ recyclisiert werden kann und bei nachfolgenden Synthesen zu den Verbindungen der Formel II wie der direkt eingesetzt werden kann. Gegebenenfalls können die Verbindungen der Formel IV vor dem Recycling gereinigt werden, z. B. in einfacher Weise durch Destillation.

Ein weiterer Vorteil dieses Verfahrens besteht in der Vermeidung des Einsatzes der Verbindungen der Formel V, 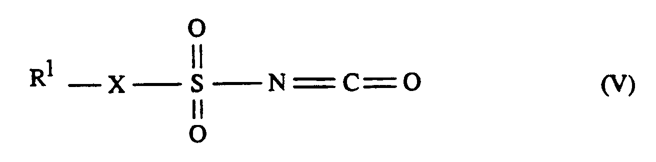 die bei herkömmlichen Verfahren zur Herstellung von Verbindungen der Formel I verwendet werden und dabei unter üblichen Reaktionstemperaturen von mehr als 110 °C intermediär gebildet werden. Unter diesen Bedingungen können die Isocyanate der Formel V jedoch thermisch sehr labil sein und teilweise zerfallen, was sich dann in niedrigen Ausbeuten widerspiegelt (s. EP-A-0131258, DE-A-2257240, G. Lohaus, Chem. Ber. 105, 2791-2799 (1972)).

Ein zusätzlicher Vorteil des erfindungsgemäßen Verfahrens besteht darin, daß die Lösungsmittel in nahezu quantitativer Ausbeute wieder recyclisiert werden können, da die Produkte der Formel I als schwerlösliche Verbindungen aus den Reaktionsmedium in hoher Reinheit und Ausbeute ausfallen. Die Lösungsmittel können anschließend z. B. durch Destillation gereinigt und dann wieder in den Prozeß eingeführt werden.

Die für die Herstellung der Verbindungen der allgemeinen Formel I nach dem erfindungsgemäßen Verfahren benötigten Ausgangsverbindungen der Formeln II und III lassen sich nach zum Teil literaturbekannten Verfahren herstellen.

So sind die Heterocyclen der Formel III entweder käuflich oder lassen sich nach geeigneten Labormethoden leicht herstellen; siehe z. B. US-A-4,310,470; EP-A-0027200; US-A-4,299,960; M. J. Langermann, C.K. Banks, J. Am. Chem. Soc. 73, 3011 (1951).

Die Verbindungen der Formel II sind neu und können analog üblichen Methoden (siehe z. B. Tietze und Eicher in "Reaktionen und Synthesen", S. 92, Thieme Verlag, Stuttgart 1981 oder Houben-Weyl, Bd. IX, S. 636) durch Umsetzung der entsprechenden Sulfonamide VI mit den entsprechenden Säurechloriden VII erhalten werden, 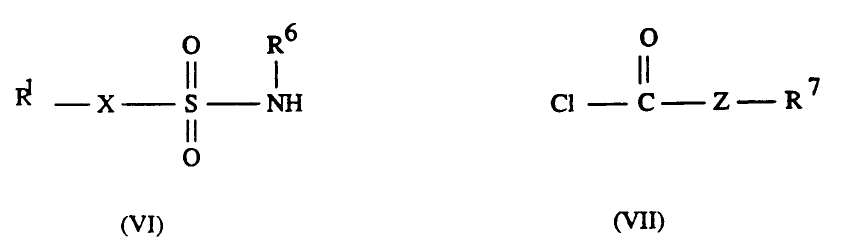 die ihrerseits wiederum nach laborüblichen Methoden zugänglich sind (siehe z. B. zu Verbindungen der Formel VI in Houben Weyl, Ergänzungsband E11, S. 1015 ff.; zu Verbindungen der Formel VII siehe z. B. W. A. Thaler, J. Chem. Soc. Chem. Communications 1968, 527).

Als überraschend ist der glatte Verlauf des erfindungsgemäßen Verfahrens und die hohe Ausbeute anzusehen, weil das Ausgangsprodukt der Formel II mehrere aktivierte, elektrophile und nucleophile Zentren enthält. Insbesondere alle der elektrophilen Zentren können prinzipiell mit den nucleophilen Substanzen der Formel III reagieren und so durch Fragmentierungsreaktionen eine Vielzahl von Nebenprodukten liefern; vgl. Beyer, Lehrbuch der Organischen Chemie, 19. Auflage, S. 128, Hirzel Verlag Stuttgart, wonach Sulfonylgruppen und Phenoxygruppen sehr gute Abgangsgruppen darstellen.

Somit stellt das erfindungsmäße Verfahren einen neuen, einfachen, auch im größeren technischen Maßstab gut reproduzierbaren und hochselektiven Prozeß zur Darstellung der Verbindungen der Formel I in nahezu quantitativen Ausbeuten dar. Das Verfahren kann kontinuierlich oder diskontinuierlich durchgeführt werden.

### BEISPIELE

### Herstellungen von 1-[(N-Methylsulfonyl-N-methyl-amino)-sulfonyl]-3-(4,6-dimethoxy-2-pyrimidyl)-harnstoff

### 1) Aus S-Benzyl-N-(N'-methyl-N'-methylsulfonyl-aminosulfonyl)-thiocarbamat

33,9 g S-Benzyl-N-(N'-methyl-N'-methylsulfonyl-aminosulfonyl)-thiocarbamat (Formel II, R¹ = CH₃, X = SO₂NCH₃, R⁶ = H, Z = S, R⁷ = Benzyl) werden zusammen mit 15,5 g 2-Amino-4,6-dimethoxypyrimidin in 60 ml Toluol 2,5 h bei 70°C gerührt. Nach Abkühlen auf 5°C wird der Niederschlag abfiltriert und mit 60 ml Toluol gewaschen und getrocknet. Man erhält 36,7 g des gewünschten Produkts der Formel (I) als weißes Pulver vom Schmelzpunkt 175-177°C. Die Reinheit ist nach HPLC-Bestimmung 97,1 Gew.-%, was einer Ausbeute von 96,6 % d. Th. entspricht.

### 2) Aus S-Methyl-N-(N'-methyl-N'-methylsulfonyl-aminosulfonyl)-thiocarbamat

26,3 g S-Methyl-N-(N'-methyl-N'-methylsulfonyl-aminosulfonyl)-thiocarbamat (Formel II, R¹ = CH₃, X = SO₂NCH₃, R⁶ = H, Z = S, R⁷ = Methyl) werden zusammen mit 15,5 g 2-Amino-4,6-dimethoxypyrimidin in 100 ml Chlorbenzol 2 h bei 75°C gerührt. Man läßt über Nacht bei Raumtemperatur stehen und filtriert den entstandenen Niederschlag ab, den man mit 30 ml Chlorbenzol wäscht. Man erhält 37,2 g des gewünschten Produkts der Formel (I) mit einer Reinheit nach HPLC von 94,6 Gew.-%, was einer Ausbeute von 95,4 % d. Th. entspricht. Der Schmelzpunkt des Produkts liegt bei 172-174°C.

### 3) Aus N-(N'-methyl-N'-methylsulfonyl-aminosulfonyl)-harnstoff

23,2 g N-(N'-methyl-N'-methylsulfonyl-aminosulfonyl)-harnstoff (Formel II, R¹ = CH₃, X = SO₂NCH₃, R⁶ = H, Z = NR⁸, R⁸ = H, R⁷ = H) werden zusammen mit 15,5 g 2-Amino-4,6-dimethoxypyrimidin in 150 ml Chlorbenzol bei 35°C unter Eingasen katalytischer Mengen Ammoniak 2 h gerührt. Anschließend wird das ausgefallene Produkt abgesaugt, mit 50 ml Chlorbenzol gewaschen und getrocknet. Man erhält 36,4 g des gewünschten Produkts der Formel (I) mit einer Reinheit nach HPLC von 96,4 Gew.-%, was einer Ausbeute von 95,1 % d. Th. entspricht. Der Schmelzpunkt des Produkts liegt bei 173-175°C.

### 4) Aus N-[(N'-methyl-N'-methylsulfonyl-aminosulfonyl)-aminocarbonyl]-morpholin

26,3 g N-[(N'-methyl-N'-methylsulfonyl-aminosulfonyl)-aminocarbonyl]-morpholin (Formel II, R¹ = CH₃, X = SO₂NCH₃, R⁶ = H, Z = NR⁸, NR⁸R⁷ = N-Morpholinyl) werden zusammen mit 15,5 g 2-Amino-4,6-dimethoxypyrimidin in 100 ml Toluol 6 h bei 75°C gerührt. Nach Abkühlen auf 5°C wird der Niederschlag abfiltriert, mit 20 ml Toluol gewaschen und getrocknet. Man erhält 35,9 g des gewünschten Produkts der Formel (I) mit einer Reinheit nach HPLC von 97,6 Gew.-%, was einer Ausbeute von 95,0 % d. Th. entspricht. Der Schmelzpunkt des Produkts liegt bei 174-175°C.

## Patentansprüche (Patentansprüche für folgende(n) Vertragsstaat(en): CH, DE, FR, GB, IT, LI, NL)

1. Verfahren zur Herstellung von Verbindungen der Formel (I) oder deren Salzen, 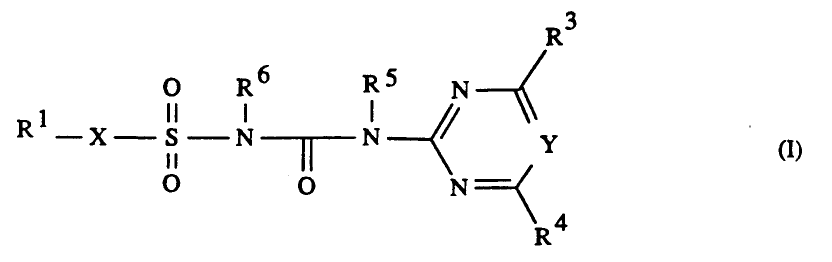 worin
X Sauerstoff , -O-NR²- oder -SO₂-NR²-,
Y Stickstoff oder CH ,
R¹ (C₁-C₆)-Alkyl, (C₂-C₆)-Alkenyl oder (C₂-C₆)-Alkinyl, wobei jeder der genannten 3 Reste unsubstituiert oder ein- oder mehrfach durch Reste aus der Gruppe Halogen, (C₁-C₄)-Alkoxy und (C₁-C₄)-Alkoxycarbonyl substituiert ist,
oder, im Falle von X = Sauerstoff, auch Phenyl, das unsubstituiert oder durch ein oder mehrere Reste aus der Gruppe Halogen, Nitro, (C₁-C₄)-Alkyl, (C₁-C₄)-Haloalkyl, (C₁-C₄)-Alkoxy, (C₁-C₄)-Haloalkoxy oder (C₁-C₄)-Alkoxycarbonyl substituiert ist,
R² Wasserstoff, (C₁-C₆)-Alkyl, (C₂-C₆)-Alkenyl, (C₂-C₆)-Alkinyl oder (C₃-C₆)-Cycloalkyl,
R³,R⁴ unabhängig voneinander Wasserstoff, (C₁-C₄)-Alkyl oder (C₁-C₄)-Alkoxy, wobei jeder der letztgenannten zwei Reste unsubstituiert oder ein- oder mehrfach durch Reste aus der Gruppe Halogen, Alkoxy und Alkylthio substituiert ist, oder Halogen, (C₁-C₄)-Alkylthio, (C₁-C₄)-Alkylamino oder Di-[(C₁-C₄)-alkyl]-amino und
R⁵,R⁶ unabhängig voneinander Wasserstoff oder (C₁-C₄)-Alkyl
bedeuten,
dadurch gekennzeichnet, daß man Verbindungen der Formel II, 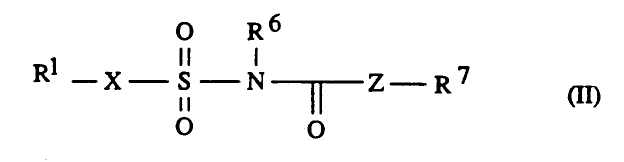 worin
Z ein Schwefelatom oder eine Gruppe der Formel -NR⁸- und
R⁷,R⁸ unabhängig voneinander Wasserstoff, (C₁-C₆)-Alkyl, Phenyl oder Benzyl, wobei die genannten aromatischen Reste unsubstituiert oder durch ein oder mehrere Reste aus der Gruppe Halogen, Alkyl und Alkoxy substituiert sind, oder im Falle Z = NR⁸ die Reste R⁷ und R⁸ auch gemeinsam mit dem Stickstoffatom einen 5-bis 7-gliedrigen Ring, der ein weiteres Stickstoff- oder Sauerstoffatom enthalten und durch einen oder mehrere Substituenten aus der Gruppe Halogen und Alkyl substituiert sein kann,
bedeuten und R¹,R⁶ und X wie in Formel I definiert sind,
mit Verbindungen der Formel III, 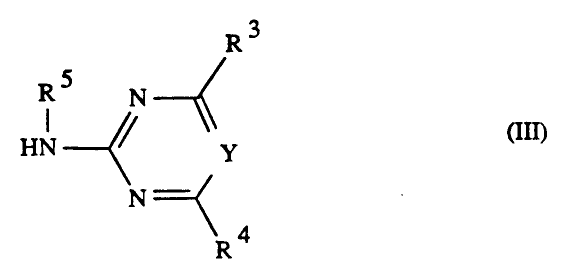 worin R³, R⁴, R⁵ und Y wie in Formel I definiert sind, umsetzt.

2. Verfahren nach Anspruch 1, dadurch gekennzeichnet, daß R¹X N-(C₁-C₆)-Alkylsulfonyl-N-(C₁-C₃)-alkyl-amino oder (C₁-C₄)-Alkoxyphenoxy, R³ und R⁴ unabhängig voneinander (C₁-C₂)-Alkyl oder (C₁-C₂)-Alkoxy und R⁵ und R⁶ Wasserstoff oder Methyl bedeuten.

3. Verfahren nach Anspruch 1 oder 2, dadurch gekennzeichnet, daß R¹X N-[(C₁-C₃)-Alkylsulfonyl]-N-[(C₁-C₂)-alkyl]-amino oder (C₁-C₃)-Alkoxyphenoxy und R³ und R⁴ unabhängig voneinander (C₁-C₂)-Alkyl oder (C₁-C₂)-Alkoxy und R⁵ und R⁶ Wasserstoff oder Methyl bedeuten.

4. Verfahren nach einem oder mehreren der Ansprüche 1 bis 3, dadurch gekennzeichnet, daß in Formel II
Z ein Schwefelatom und R⁷ (C₁-C₄)-Alkyl, Phenyl oder Benzyl, wobei die genannten aromatischen Reste unsubstituiert oder durch ein oder mehrere Reste aus der Gruppe Halogen, (C₁-C₂)-Alkyl und (C₁-C₂)-Alkoxy substituiert sind, bedeuten und R¹,R⁶ und X wie in Formel I definiert sind.

5. Verfahren nach einem oder mehreren der Ansprüche 1 bis 3, dadurch gekennzeichnet, daß in Formel II
Z eine Gruppe der Formel -NR⁸-, R⁷ Wasserstoff oder (C₁-C₄)-Alkyl, Phenyl oder Benzyl, wobei die genannten aromatischen Reste unsubstituiert oder durch ein oder mehrere Reste aus der Gruppe Halogen, (C₁-C₂)-Alkyl und (C₁-C₂)-Alkoxy substituiert sind und R⁸ Wasserstoff oder (C₁-C₄)-Alkyl, oder
R⁷,R⁸ gemeinsam mit dem Stickstoffatom einen 5- bis 7-gliedrigen Ring, der ein weiteres Stickstoff- oder Sauerstoffatom enthalten und durch einen oder mehrere Substituenten aus der Gruppe Halogen, (C₁-C₄)-Alkyl substituiert sein kann,
bedeuten und R¹,R⁶ und X wie in Formel I definiert sind.

6. Verfahren nach einem oder mehreren der Ansprüche 1 bis 5, dadurch gekennzeichnet, daß man das Verfahren in Gegenwart eines unter den Reaktionsbedingungen inerten anorganischen oder organischen Lösungsmittels oder eines Gemisches mehrerer entsprechender Lösungsmittel durchführt.

7. Verfahren nach einem oder mehreren der Ansprüche 1 bis 6, dadurch gekennzeichnet, daß das Verfahren in Gegenwart eines inerten organischen Lösungsmittels durchgeführt wird.

8. Verfahren nach einem oder mehreren der Ansprüche 1 bis 7, dadurch gekennzeichnet, daß die Verbindung der Formel II im Verhältnis zur Verbindung der Formel III äquimolar oder in geringem Überschuß eingesetzt wird.

9. Verfahren nach Anspruch 8, dadurch gekennzeichnet, daß das Molverhältnis II:III im Bereich von 1:1 bis 1,2:1 ist.

10. Verfahren nach einem oder mehreren der Ansprüche 1 bis 9, dadurch gekennzeichnet, daß die Reaktionstemperaturen im Bereich von 0°C bis 140°C sind.

11. Verbindungen der Formel II, 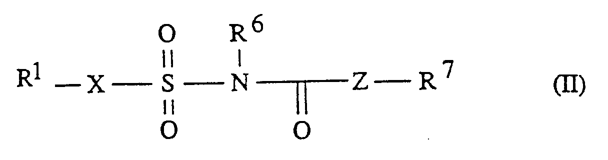 worin R¹, R⁶, R⁷, X und Z wie in Formel II nach einem oder mehreren der Ansprüche 1 bis 4 oder 1 bis 3 und 5 definiert sind.

12. Verfahren zur Herstellung von Verbindungen der Formel II nach Anspruch 11, dadurch gekennzeichnet, daß man Sulfonamide der Formel VI mit Säurechloriden der Formel VII, 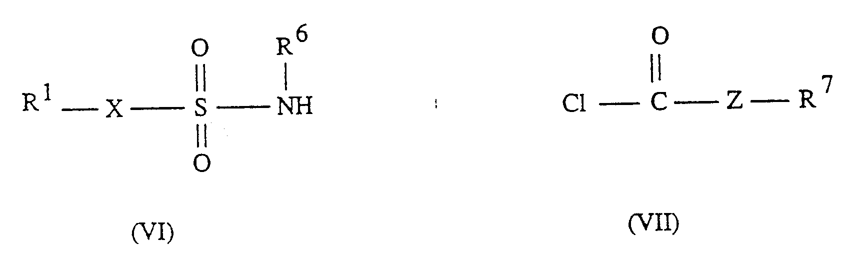 worin R¹, R⁶, R⁷, X und Z wie in Formel II definiert sind, umsetzt.

13. Verwendung von Verbindungen der Formel II nach Anspruch 11 zur Herstellung von Sulfonylharnstoffen.

## Patentansprüche (Patentansprüche für folgende(n) Vertragsstaat(en): ES)

1. Verfahren zur Herstellung von Verbindungen der Formel (I) oder deren Salzen, 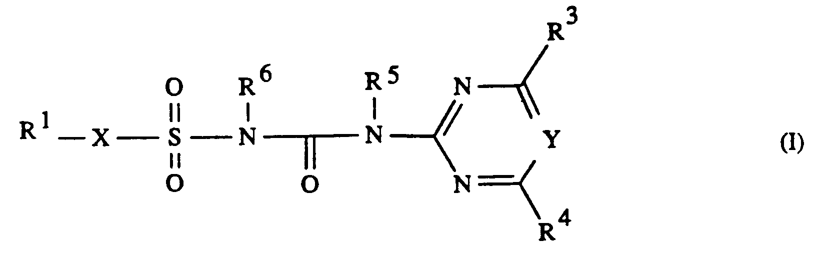 worin
X Sauerstoff , -O-NR²- oder -SO₂-NR²-,
Y Stickstoff oder CH ,
R¹ (C₁-C₆)-Alkyl, (C₂-C₆)-Alkenyl oder (C₂-C₆)-Alkinyl, wobei jeder der genannten 3 Reste unsubstituiert oder ein- oder mehrfach durch Reste aus der Gruppe Halogen, (C₁-C₄)-Alkoxy und (C₁-C₄)-Alkoxycarbonyl substituiert ist,
oder, im Falle von X = Sauerstoff, auch Phenyl, das unsubstituiert oder durch ein oder mehrere Reste aus der Gruppe Halogen, Nitro, (C₁-C₄)-Alkyl, (C₁-C₄)-Haloalkyl, (C₁-C₄)-Alkoxy, (C₁-C₄)-Haloalkoxy oder (C₁-C₄)-Alkoxycarbonyl substituiert ist,
R² Wasserstoff, (C₁-C₆)-Alkyl, (C₂-C₆)-Alkenyl, (C₂-C₆)-Alkinyl oder (C₃-C₆)-Cycloalkyl,
R³,R⁴ unabhängig voneinander Wasserstoff, (C₁-C₄)-Alkyl oder (C₁-C₄)-Alkoxy, wobei jeder der letztgenannten zwei Reste unsubstituiert oder ein- oder mehrfach durch Reste aus der Gruppe Halogen, Alkoxy und Alkylthio substituiert ist, oder Halogen, (C₁-C₄)-Alkylthio, (C₁-C₄)-Alkylamino oder Di-[(C₁-C₄)-alkyl]-amino und
R⁵,R⁶ unabhängig voneinander Wasserstoff oder (C₁-C₄)-Alkyl
bedeuten,
dadurch gekennzeichnet, daß man Verbindungen der Formel II, 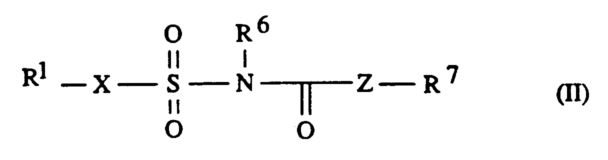 worin
Z ein Schwefelatom oder eine Gruppe der Formel -NR⁸- und
R⁷,R⁸ unabhängig voneinander Wasserstoff, (C₁-C₆)-Alkyl, Phenyl oder Benzyl, wobei die genannten aromatischen Reste unsubstituiert oder durch ein oder mehrere Reste aus der Gruppe Halogen, Alkyl und Alkoxy substituiert sind, oder im Falle Z = NR⁸ die Reste R⁷ und R⁸ auch gemeinsam mit dem Stickstoffatom einen 5- bis 7-gliedrigen Ring, der ein weiteres Stickstoff- oder Sauerstoffatom enthalten und durch einen oder mehrere Substituenten aus der Gruppe Halogen und Alkyl substituiert sein kann,
bedeuten und R¹,R⁶ und X wie in Formel I definiert sind,
mit Verbindungen der Formel III, 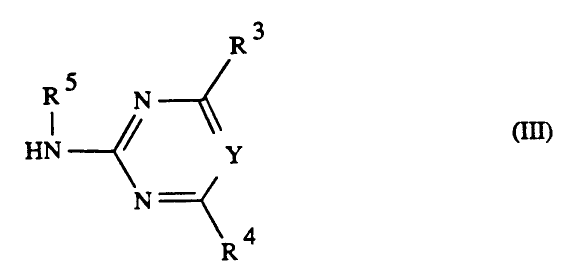 worin R³, R⁴, R⁵ und Y wie in Formel I definiert sind, umsetzt.

2. Verfahren nach Anspruch 1, dadurch gekennzeichnet, daß R¹X N-(C₁-C₆)-Alkylsulfonyl-N-(C₁-C₃)-alkyl-amino oder (C₁-C₄)-Alkoxyphenoxy, R³ und R⁴ unabhängig voneinander (C₁-C₂)-Alkyl oder (C₁-C₂)-Alkoxy und R⁵ und R⁶ Wasserstoff oder Methyl bedeuten.

3. Verfahren nach Anspruch 1 oder 2, dadurch gekennzeichnet, daß R¹X N-[(C₁-C₃)-Alkylsulfonyl]-N-[(C₁-C₂)-alkyl]-amino oder (C₁-C₃)-Alkoxyphenoxy und R³ und R⁴ unabhängig voneinander (C₁-C₂)-Alkyl oder (C₁-C₂)-Alkoxy und R⁵ und R⁶ Wasserstoff oder Methyl bedeuten.

4. Verfahren nach einem oder mehreren der Ansprüche 1 bis 3, dadurch gekennzeichnet, daß in Formel II
Z ein Schwefelatom und R⁷ (C₁-C₄)-Alkyl, Phenyl oder Benzyl, wobei die genannten aromatischen Reste unsubstituiert oder durch ein oder mehrere Reste aus der Gruppe Halogen, (C₁-C₂)-Alkyl und (C₁-C₂)-Alkoxy substituiert sind, bedeuten und R¹,R⁶ und X wie in Formel I definiert sind.

5. Verfahren nach einem oder mehreren der Ansprüche 1 bis 3, dadurch gekennzeichnet, daß in Formel II
Z eine Gruppe der Formel -NR⁸-, R⁷ Wasserstoff oder (C₁-C₄)-Alkyl, Phenyl oder Benzyl, wobei die genannten aromatischen Reste unsubstituiert oder durch ein oder mehrere Reste aus der Gruppe Halogen, (C₁-C₂)-Alkyl und (C₁-C₂)-Alkoxy substituiert sind und R⁸ Wasserstoff oder (C₁-C₄)-Alkyl, oder
R⁷,R⁸ gemeinsam mit dem Stickstoffatom einen 5- bis 7-gliedrigen Ring, der ein weiteres Stickstoff- oder Sauerstoffatom enthalten und durch einen oder mehrere Substituenten aus der Gruppe Halogen, (C₁-C₄)-Alkyl substituiert sein kann,
bedeuten und R¹,R⁶ und X wie in Formel I definiert sind.

6. Verfahren nach einem oder mehreren der Ansprüche 1 bis 5, dadurch gekennzeichnet, daß man das Verfahren in Gegenwart eines unter den Reaktionsbedingungen inerten anorganischen oder organischen Lösungsmittels oder eines Gemisches mehrerer entsprechender Lösungsmittel durchführt.

7. Verfahren nach einem oder mehreren der Ansprüche 1 bis 6, dadurch gekennzeichnet, daß das Verfahren in Gegenwart eines inerten organischen Lösungsmittels durchgeführt wird.

8. Verfahren nach einem oder mehreren der Ansprüche 1 bis 7, dadurch gekennzeichnet, daß die Verbindung der Formel II im Verhältnis zur Verbindung der Formel III äquimolar oder in geringem Überschuß emgesetzt wird.

9. Verfahren nach Anspruch 8, dadurch gekennzeichnet, daß das Molverhältnis II:III im Bereich von 1:1 bis 1,2:1 ist.

10. Verfahren nach einem oder mehreren der Ansprüche 1 bis 9, dadurch gekennzeichnet, daß die Reaktionstemperaturen im Bereich von 0°C bis 140°C sind.

11. Verfahren zur Herstellung von Verbindungen der Formel II, 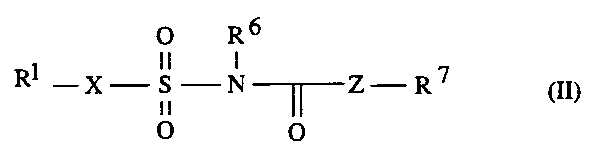 worin R¹, R⁶, R⁷, X und Z wie in Formel II nach einem oder mehreren der Ansprüche 1 bis 4 oder 1 bis 3 und 5 definiert sind, dadurch gekennzeichnet, daß man Sulfonamide der Formel VI mit Säurechloriden der Formel VII, 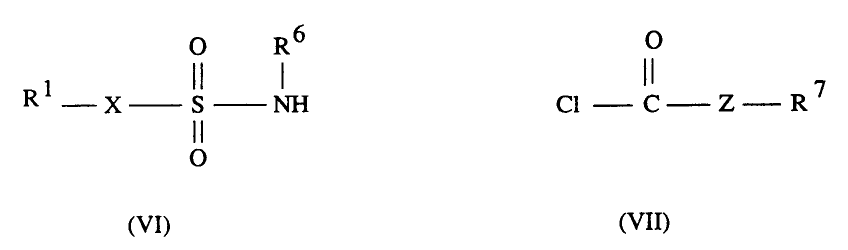 worin R¹, R⁶, R⁷, X und Z wie in Formel II definiert sind, umsetzt.

12. Verwendung von Verbindungen der Formel II, wie sie in Anspruch 11 definiert sind, zur Herstellung von Sulfonylharnstoffen.

## Claims (Claims for the following Contracting State(s): CH, DE, FR, GB, IT, LI, NL)

1. A process for the preparation of a compound of the formula (I) or a salt thereof 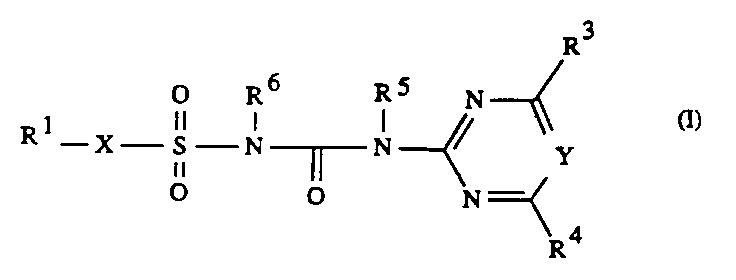 where
X is oxygen, -O-NR²- or -SO₂-NR²,
Y is nitrogen or CH,
R¹ is (C₁-C₆)-alkyl, (C₂-C₆)-alkenyl or (C₂-C₆)-alkynyl, each of the 3 radicals mentioned being unsubstituted or mono- or polysubstituted by radicals selected from the group consisting of halogen, (C₁-C₄)-alkoxy and (C₁-C₄)-alkoxycarbonyl,
or, in the event that X = oxygen, also phenyl which is unsubstituted or substituted by one or more radicals selected from the group consisting of halogen, nitro, (C₁-C₄)-alkyl, (C₁-C₄)-haloalkyl, (C₁-C₄)-alkoxy, (C₁-C₄)-haloalkoxy or (C₁-C₄)-alkoxycarbonyl,
R² is hydrogen, (C₁-C₆)-alkyl, (C₂-C₆)-alkenyl, (C₂-C₆)-alkynyl or (C₃-C₆)-cycloalkyl,
R³,R⁴ independently of one another are hydrogen, (C₁-C₄)-alkyl or (C₁-C₄)-alkoxy, each of the last-mentioned two radicals being unsubstituted or mono- or polysubstituted by radicals selected from the group consisting of halogen, alkoxy and alkylthio, or halogen, (C₁-C₄)-alkylthio, (C₁-C₄)-alkylamino or di[(C₁-C₄)-alkyl]amino and
R⁵,R⁶ independently of one another are halogen or (C₁-C₄)-alkyl,
which comprises compounds of the formula II 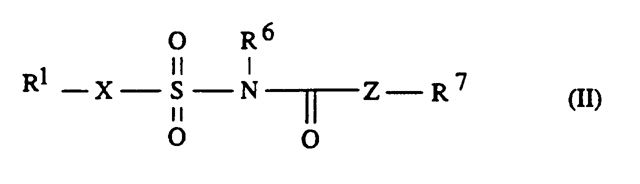 where
Z is a sulfur atom or a group of the formula -NR⁸- and
R⁷,R⁸ independently of one another are hydrogen, (C₁-C₆)-alkyl, phenyl or benzyl, the abovementioned aromatic radicals being unsubstituted or substituted by one or more radicals selected from the group consisting of halogen, alkyl and alkoxy,
or, in the event that Z = NR⁸, the radicals R⁷ and R⁸ also together with the nitrogen atom are a 5- to 7-membered ring which can contain a further nitrogen or oxygen atom and which can be substituted by one or more substituents selected from the group consisting of halogen and alkyl
and R¹,R⁶ and X are as defined in formula I
are reacted with compounds of the formula III 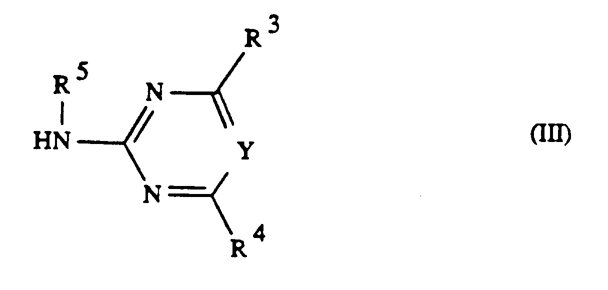 where R³,R⁴,R⁵ and Y are as defined in formula I.

2. The process as claimed in claim 1, wherein R¹X is N-(C₁-C₆)-alkylsulfonyl-N-(C₁-C₃)-alkylamino or (C₁-C₄)-alkoxyphenoxy, R³ and R⁴ independently of one another are (C₁-C₂)-alkyl or (C₁-C₂)-alkoxy and R⁵ and R⁶ are hydrogen or methyl.

3. The process as claimed in claim 1 or 2, wherein R¹X is N-[(C₁-C₃)-alkylsulfony]-N-[(C₁-C₂)-alkyl]amino or (C₁-C₃)-alkoxyphenoxy and R³ and R⁴ independently of one another are (C₁-C₂)-alkyl or (C₁-C₂)-alkoxy and R⁵ and R⁶ are hydrogen or methyl.

4. The process as claimed in one or more of claims 1 to 3, wherein, in formula II,
Z is a sulfur atom and R⁷ is (C₁-C₄)-alkyl, phenyl or benzyl, the abovementioned aromatic radicals being unsubstituted or substituted by one or more radicals selected from the group consisting of halogen, (C₁-C₂)-alkyl and (C₁-C₂)-alkoxy, and R¹,R⁶ and X are as defined in formula I.

5. The process as claimed in one or more of claims 1 to 3, wherein, in formula II
Z is a group of the formula -NR⁸- and R⁷ is hydrogen or (C₁-C₄)-alkyl, phenyl or benzyl, the above-mentioned aromatic radicals being unsubstituted or substituted by one or more radicals selected from the group consisting of halogen, (C₁-C₂)-alkyl and (C₁-C₂)-alkoxy and R⁸ is hydrogen or (C₁-C₄)-alkyl, or
R⁷, R⁸ together with the nitrogen atom are a 5- to 7-membered ring which can contain a further nitrogen or oxygen atom and which can be substituted by one or more substituents selected from the group consisting of halogen and (C₁-C₄)-alkyl,
and R¹,R⁶ and X are as defined in formula I.

6. The process as claimed in one or more of claims 1 to 5, which is carried out in the presence of an inorganic or organic solvent which is inert under the reaction conditions or of a mixture of a plurality of such solvents.

7. The process as claimed in one or more of claims 1 to 6, which is carried out in the presence of an inert organic solvent.

8. The process as claimed in one or more of claims 1 to 7, wherein the compound of the formula II is employed in equimolar amounts or in a small excess, relative to the compound of the formula III.

9. The process as claimed in claim 8, wherein the molar ratio of II:III is in a range from 1:1 to 1.2:1.

10. The process as claimed in one or more of claims 1 to 9, wherein the reaction temperatures are in a range from 0°C to 140°C.

11. A compound of the formula II 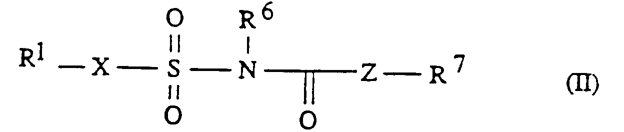 where R¹,R⁶,R⁷, X and Z are as defined in formula II as claimed in one or more of claims 1 to 4 or 1 to 3 and 5.

12. A process for the preparation of a compound of the formula II as claimed in claim 11, which comprises reacting sulfonamides of the formula VI with acid chlorides of the formula VII 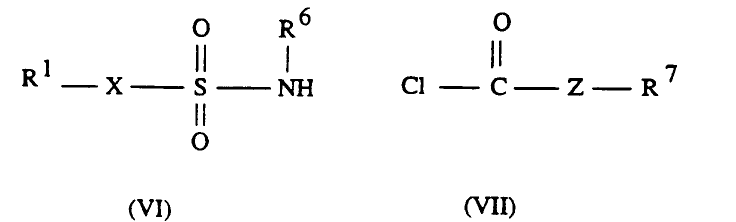 in which R¹,R⁶,R⁷, X and Z are as defined in formula II.

13. The use of a compound of the formula II as claimed in claim 11 for the preparation of sulfonylureas.

## Claims (Claims for the following Contracting State(s): ES)

1. A process for the preparation of a compound of the formula (I) or a salt thereof 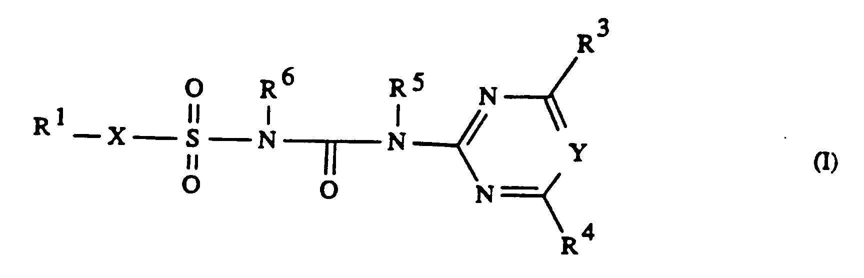 where
X is oxygen, -O-NR²- or -SO₂-NR²,
Y is nitrogen or CH,
R¹ is (C₁-C₆)-alkyl, (C₂-C₆)-alkenyl or (C₂-C₆)-alkynyl, each of the 3 radicals mentioned being unsubstituted or mono- or polysubstituted by radicals selected from the group consisting of halogen, (C₁-C₄)-alkoxy and (C₁-C₄)-alkoxycarbonyl,
or, in the event that X = oxygen, also phenyl which is unsubstituted or substituted by one or more radicals selected from the group consisting of halogen, nitro, (C₁-C₄)-alkyl, (C₁-C₄)-haloalkyl, (C₁-C₄)-alkoxy, (C₁-C₄)-haloalkoxy or (C₁-C₄)-alkoxycarbonyl,
R² is hydrogen, (C₁-C₆)-alkyl, (C₂-C₆)-alkenyl, (C₂-C₆)-alkynyl or (C₃-C₆)-cycloalkyl,
R³,R⁴ independently of one another are hydrogen, (C₁-C₄)-alkyl or (C₁-C₄)-alkoxy, each of the last-mentioned two radicals being unsubstituted or mono- or polysubstituted by radicals selected from the group consisting of halogen, alkoxy and alkylthio, or halogen, (C₁-C₄)-alkylthio, (C₁-C₄)-alkylamino or di[(C₁-C₄)-alkyl]amino and
R⁵,R⁶ independently of one another are halogen or (C₁-C₄)-alkyl,
which comprises compounds of the formula II 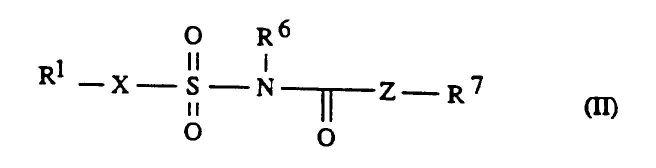 where
Z is a sulfur atom or a group of the formula -NR⁸- and
R⁷,R⁸ independently of one another are hydrogen, (C₁-C₆)-alkyl, phenyl or benzyl, the abovementioned aromatic radicals being unsubstituted or substituted by one or more radicals selected from the group consisting of halogen, alkyl and alkoxy,
or, in the event that Z = NR⁸, the radicals R⁷ and R⁸ also together with the nitrogen atom are a 5- to 7-membered ring which can contain a further nitrogen or oxygen atom and which can be substituted by one or more substituents selected from the group consisting of halogen and alkyl
and R¹,R⁶ and X are as defined in formula I
are reacted with compounds of the formula III 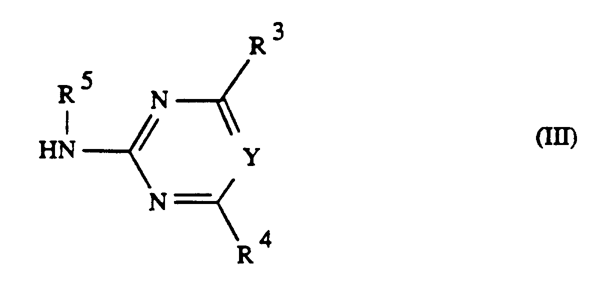 where R³,R⁴,R⁵ and Y are as defined in formula I.

2. The process as claimed in claim 1, wherein R¹X is N-(C₁-C₆)-alkylsulfonyl-N-(C₁-C₃)-alkylamino or (C₁-C₄)-alkoxyphenoxy, R³ and R⁴ independently of one another are (C₁-C₂)-alkyl or (C₁-C₂)-alkoxy and R⁵ and R⁶ are hydrogen or methyl.

3. The process as claimed in claim 1 or 2, wherein R¹X is N-[(C₁-C₃)-alkylsulfony]-N-[(C₁-C₂)-alkyl]amino or (C₁-C₃)-alkoxyphenoxy and R³ and R⁴ independently of one another are (C₁-C₂)-alkyl or (C₁-C₂)-alkoxy and R⁵ and R⁶ are hydrogen or methyl.

4. The process as claimed in one or more of claims 1 to 3, wherein, in formula II,
Z is a sulfur atom and R⁷ is (C₁-C₄)-alkyl, phenyl or benzyl, the abovementioned aromatic radicals being unsubstituted or substituted by one or more radicals selected from the group consisting of halogen, (C₁-C₂)-alkyl and (C₁-C₂)-alkoxy, and R¹,R⁶ and X are as defined in formula I.

5. The process as claimed in one or more of claims 1 to 3, wherein, in formula II
Z is a group of the formula -NR⁸- and R⁷ is hydrogen or (C₁-C₄)-alkyl, phenyl or benzyl, the above-mentioned aromatic radicals being unsubstituted or substituted by one or more radicals selected from the group consisting of halogen, (C₁-C₂)-alkyl and (C₁-C₂)-alkoxy and R⁸ is hydrogen or (C₁-C₄)-alkyl, or
R⁷, R⁸ together with the nitrogen atom are a 5- to 7-membered ring which can contain a further nitrogen or oxygen atom and which can be substituted by one or more substituents selected from the group consisting of halogen and (C₁-C₄)-alkyl,
and R¹,R⁶ and X are as defined in formula I.

6. The process as claimed in one or more of claims 1 to 5, which is carried out in the presence of an inorganic or organic solvent which is inert under the reaction conditions or of a mixture of a plurality of such solvents.

7. The process as claimed in one or more of claims 1 to 6, which is carried out in the presence of an inert organic solvent.

8. The process as claimed in one or more of claims 1 to 7, wherein the compound of the formula II is employed in equimolar amounts or in a small excess, relative to the compound of the formula III.

9. The process as claimed in claim 8, wherein the molar ratio of II:III is in a range from 1:1 to 1.2:1.

10. The process as claimed in one or more of claims 1 to 9, wherein the reaction temperatures are in a range from 0°C to 140°C.

11. A process for the preparation of a compound of the formula II 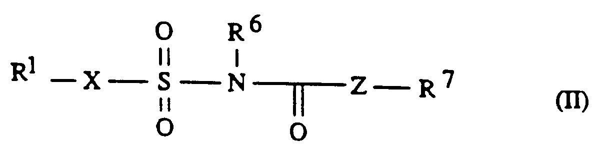 where R¹,R⁶,R⁷, X and Z are as defined in formula II as claimed in one or more of claims 1 to 4 or 1 to 3 and 5, which comprises reacting sulfonamides of the formula VI with acid chlorides of the formula VII 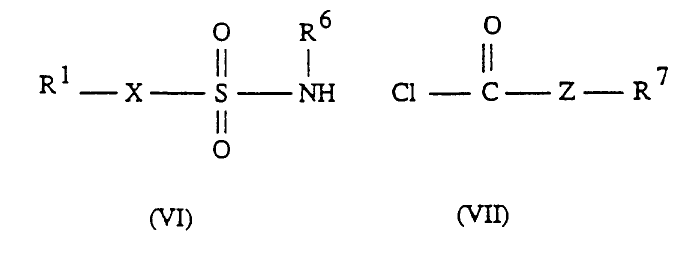 in which R¹,R⁶,R⁷, X and Z are as defined in formula II.

12. The use of a compound of the formula II as defined in claim 11 for the preparation of sulfonylureas.

## Revendications (Revendications pour l'(les) Etat(s) contractant(s) suivant(s): CH, DE, FR, GB, IT, LI, NL)

1. Procédé de préparation de composés de formule (I) ou de sels de ceux-ci : 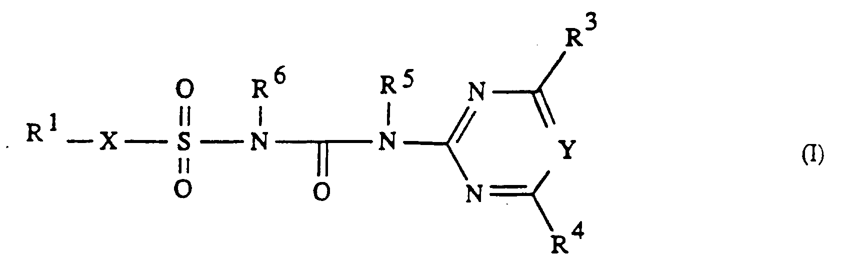 dans laquelle
X représente un atome d'oxygène, -O-NR²- ou -SO₂-NR²-,
Y représente un atome d'azote ou CH,
R¹ représente un groupe alkyle en C₁-C₆, alcényle en C₂-C₆ ou alcynyle en C₂-C₆, chacun des 3 radicaux cités étant non substitué ou une ou plusieurs fois substitué par des résidus choisis parmi un atome d'halogène, un groupe alkoxy en C₁-C₄ et alkoxycarbonyle en C₁-C₄,
ou, dans le cas où X = un atome d'azote, également un groupe phényle qui est non substitué ou substitué par un ou plusieurs groupes choisis parmi un atome d'halogène, un groupe nitro, alkyle en C₁-C₄, haloalkyle en C₁-C₄, alkoxy en C₁-C₄, haloalkoxy en C₁-C₄ ou alkoxycarbonyle en C₁-C₄,
R² représente un atome d'hydrogène, un groupe alkyle en C₁-C₆, alcényle en C₂-C₆, alcynyle en C₂-C₆ ou cycloalkyle en C₃-C₆,
R³, R⁴ représentent indépendamment l'un de l'autre un atome d'hydrogène, ou un groupe alkyle en C₁-C₄ ou alkoxy en C₁-C₄, chacun des deux derniers radicaux cités étant non substitué ou une ou plusieurs fois substitué par des résidus choisis parmi un atome d'halogène, un groupe alkoxy et alkylthio, ou représentent un atome d'halogène, un groupe alkylthio en C₁-C₄, alkylamino en C₁-C₄ ou di(alkyl en C₁-C₄)-amino et
R⁵, R⁶ représentent indépendamment l'un de l'autre un atome d'hydrogène ou un groupe alkyle en C₁-C₄,
caractérisé en ce que, l'on fait réagir des composés de formule II, 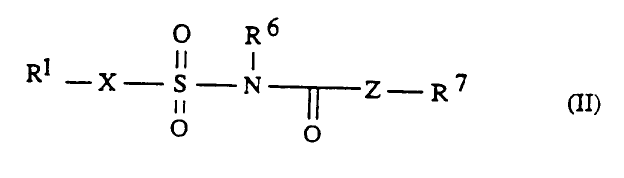 dans laquelle
Z représente un atome de soufre ou un groupe de formule -NR⁸- et
R⁷, R⁸ représentent indépendamment l'une de l'autre un atome d'hydrogène, un groupe alkyle en C₁-C₆, phényle ou benzyle, les radicaux aromatiques cités étant non substitués ou substitués par un ou plusieurs radicaux choisis parmi un atome d'halogène, un groupe alkyle et alkoxy, ou dans le cas où Z = NR⁸ les radicaux R⁷ et R⁸ représentent également ensemble avec l'atome d'azote un cycle à 5 à 7 chaînons, qui peut contenir un autre atome d'azote ou d'oxygène et être substitué par un ou plusieurs substituants choisis entre un atome d'halogène et un groupe alkyle,
et R¹, R⁶ et X sont définis comme dans la formule I,
avec des composés de formule III, 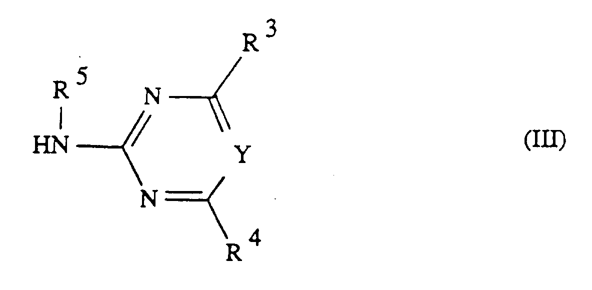 dans laquelle R³, R⁴, R⁵ et Y sont définis comme dans la formule I.

2. Procédé selon la revendication 1, caractérisé en ce que, R¹X représente un radical N-(alkyl en C₁-C₆)sulfonyl-N-(alkyl en C₁-C₃)-amino ou (alkoxy en C₁-C₄)phénoxy, R³ et R⁴ représentent indépendamment l'un de l'autre un groupe alkyle en C₁-C₂ ou alkoxy en C₁-C₂ et R⁵ et R⁶ représentent un atome d'hydrogène ou un groupe méthyle.

3. Procédé selon la revendication 1 ou 2, caractérisé en ce que, R¹X représente un groupe N-[alkylsulfonyle en C₁-C₃]-N-(alkyl en C₁-C₂)-amino ou (alkoxy en C₁-C₃)phénoxy et R³ et R⁴ représentent indépendamment l'un de l'autre un groupe alkyle en C₁-C₂ ou alkoxy en C₁-C₂ et R⁵ et R⁶ représentent un atome d'hydrogène ou un groupe méthyle.

4. Procédé selon une ou plusieurs des revendications 1 à 3, caractérisé en ce que, dans la formule II
Z représente un atome de soufre et R⁷ un groupe alkyle en C₁-C₄, phényle ou benzyle, les radicaux aromatiques cités étant non substitués ou substitués par un ou plusieurs restes choisis parmi un atome d'halogène, un groupe alkyle en C₁-C₂ et alkoxy en C₁-C₂ et R¹, R⁶ et X sont définis comme dans la formule I.

5. Procédé selon une ou plusieurs des revendications 1 à 3, caractérisé en ce que, dans la formule II
Z représente un groupe de formule -NR⁸-, R⁷ un atome d'hydrogène ou un groupe alkyle en C₁-C₄, phényle ou benzyle, les radicaux aromatiques cités étant non substitués ou substitués par un ou plusieurs restes choisis parmi un atome d'halogène, un groupe alkyle en C₁-C₂ et alkoxy en C₁-C₂ et R⁸ représente un atome d'hydrogène ou un groupe alkyle en C₁-C₄, ou
R⁷, R⁸ représentent ensemble avec l'atome d'azote un cycle à 5 à 7 chaînons, qui peut contenir un autre atome d'azote ou d'oxygène et être substitué par un ou plusieurs substituants choisis entre un atome d'halogène, un groupe alkyle en C₁-C₄,
et R¹, R⁶ et X sont définis comme dans la formule I.

6. Procédé selon une ou plusieurs des revendications 1 à 5, caractérisé en ce qu'on réalise le procédé en présence d'un solvant organique ou inorganique inerte dans les conditions de réaction ou d'un mélange de plusieurs solvants correspondants.

7. Procédé selon une ou plusieurs des revendications 1 à 6, caractérisé en ce le procédé est réalisé en présence d'un solvant organique inerte.

8. Procédé selon une ou plusieurs des revendications 1 à 7, caractérisé en ce que l'on fait réagir le composé de formule II en quantité équimolaire ou en léger excès par rapport au composé de formule III.

9. Procédé selon la revendication 8, caractérisé en ce que le rapport molaire II:III est d'environ 1:1 à 1,2:1.

10. Procédé selon une ou plusieurs des revendications 1 à 9, caractérisé en ce que les températures de réaction sont d'environ 0°C à 140°C.

11. Composés de formule II, 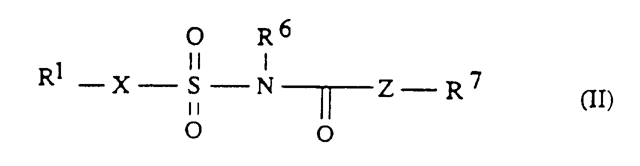 dans laquelle R¹, R⁶, R⁷, X et Z sont définis comme dans la formule II selon une ou plusieurs des revendications 1 à 4 ou 1 à 3 et 5.

12. Procédé de préparation de composés de formule II selon la revendication 11, caractérisé en ce que, l'on fait réagir des sulfonamides de formule VI avec des chlorures d'acides de formule VII, 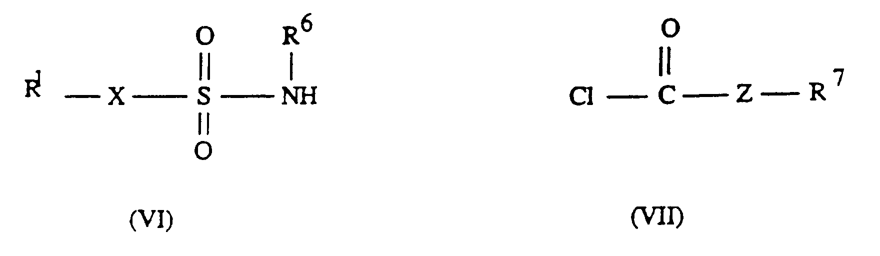 dans laquelle R¹, R⁶, R⁷, X et Z sont définis comme dans la formule II.

13. Utilisation de composés de formule II selon la revendication 11 pour la préparation de sulfonylurées.

## Revendications (Revendications pour l'(les) Etat(s) contractant(s) suivant(s): ES)

1. Procédé de préparation de composés de formule (I) ou de sels de ceux-ci : 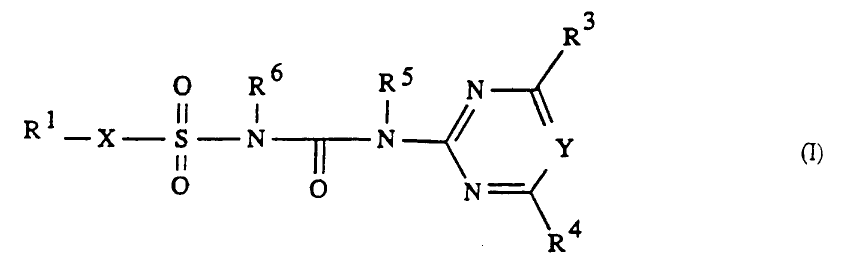 dans laquelle
X représente un atome d'oxygène, -O-NR²- ou -SO₂-NR²-,
Y représente un atome d'azote ou CH,
R¹ représente un groupe alkyle en C₁-C₆, alcényle en C₂-C₆ ou alcynyle en C₂-C₆, chacun des 3 radicaux cités étant non substitué ou une ou plusieurs fois substitué par des résidus choisis parmi un atome d'halogène, un groupe alkoxy en C₁-C₄ et alkoxycarbonyle en C₁-C₄,
ou, dans le cas où X = un atome d'azote, également un groupe phényle qui est non substitué ou substitué par un ou plusieurs groupes choisis parmi un atome d'halogène, un groupe nitro, alkyle en C₁-C₄, haloalkyle en C₁-C₄, alkoxy en C₁-C₄, haloalkoxy en C₁-C₄ ou alkoxycarbonyle en C₁-C₄,
R² représente un atome d'hydrogène, un groupe alkyle en C₁-C₆, alcényle en C₂-C₆, alcynyle en C₂-C₆ ou cycloalkyle en C₃-C₆,
R³, R⁴ représentent indépendamment l'un de l'autre un atome d'hydrogène, ou un groupe alkyle en C₁-C₄ ou alkoxy en C₁-C₄, chacun des deux derniers radicaux cités étant non substitué ou une ou plusieurs fois substitué par des résidus choisis parmi un atome d'halogène, un groupe alkoxy et alkylthio, ou représentent un atome d'halogène, un groupe alkylthio en C₁-C₄, alkylamino en C₁-C₄ ou di(alkyle en C₁-C₄)-amino et
R⁵, R⁶ représentent indépendamment l'un de l'autre un atome d'hydrogène ou un groupe alkyle en C₁-C₄,
caractérisé en ce que, l'on fait réagir des composés de formule II, 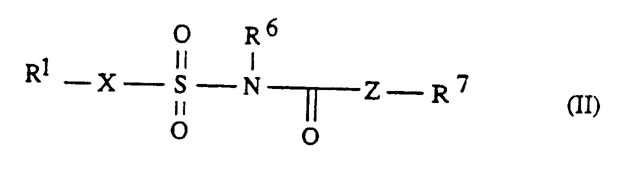 dans laquelle
Z représente un atome de soufre ou un groupe de formule -NR⁸- et
R⁷, R⁸ représentent indépendamment l'une de l'autre un atome d'hydrogène, un groupe alkyle en C₁-C₆, phényle ou benzyle, les radicaux aromatiques cités étant non substitués ou substitués par un ou plusieurs radicaux choisis parmi un atome d'halogène, un groupe alkyle et alkoxy, ou dans le cas où Z = NR⁸ les radicaux R⁷ et R⁸ représentent également ensemble avec l'atome d'azote un cycle à 5 à 7 chaînons, qui peut contenir un autre atome d'azote ou d'oxygène et être substitué par un ou plusieurs substituants choisis entre un atome d'halogène et un groupe alkyle,
et R¹, R⁶ et X sont définis comme dans la formule I,
avec des composés de formule III, 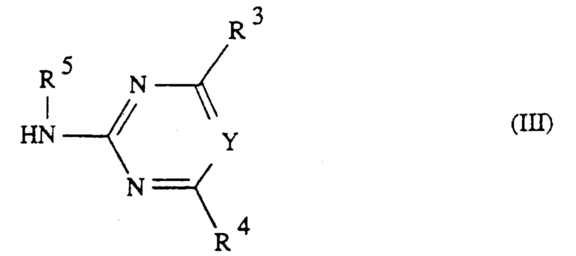 dans laquelle R³, R⁴, R⁵ et Y sont définis comme dans la formule I.

2. Procédé selon la revendication 1, caractérisé en ce que, R¹X représente un radical N-(alkylsulfonyle en C₁-C₆)-N-(alkyl en C₁-C₃)-amino ou (alkoxy en C₁-C₄)phénoxy, R³ et R⁴ représentent indépendamment l'un de l'autre un groupe alkyle en C₁-C₂ ou alkoxy en C₁-C₂ et R⁵ et R⁶ représentent un atome d'hydrogène ou un groupe méthyle.

3. Procédé selon la revendication 1 ou 2, caractérisé en ce que, R¹X représente un groupe N-[alkylsulfonyle en C₁-C₃]-N-(alkyl en C₁-C₂)-amino ou (alkoxy en C₁-C₃)phénoxy et R³ et R⁴ représentent indépendamment l'un de l'autre un groupe alkyle en C₁-C₂ ou alkoxy en C₁-C₂ et R⁵ et R⁶ représentent un atome d'hydrogène ou un groupe méthyle.

4. Procédé selon une ou plusieurs des revendications 1 à 3, caractérisé en ce que, dans la formule II
Z représente un atome de soufre et R⁷ un groupe alkyle en C₁-C₄, phényle ou benzyle, les radicaux aromatiques cités étant non substitués ou substitués par un ou plusieurs restes choisis parmi un atome d'halogène, un groupe alkyle en C₁-C₂ et alkoxy en C₁-C₂ et R¹, R⁶ et X sont définis comme dans la formule I.

5. Procédé selon une ou plusieurs des revendications 1 à 3, caractérisé en ce que, dans la formule II
Z représente un groupe de formule -NR⁸-, R⁷ un atome d'hydrogène ou un groupe alkyle en C₁-C₄, phényle ou benzyle, les radicaux aromatiques cités étant non substitués ou substitués par un ou plusieurs restes choisis parmi un atome d'halogène, un groupe alkyle en C₁-C₂ et alkoxy en C₁-C₂ et R⁸ représente un atome d'hydrogène ou un groupe alkyle en C₁-C₄, ou
R⁷, R⁸ représentent ensemble avec l'atome d'azote un cycle à 5 à 7 chaînons, qui peut contenir un autre atome d'azote ou d'oxygène et être substitué par un ou plusieurs substituants choisis entre un atome d'halogène, un groupe alkyle en C₁-C₄,
et R¹, R⁶ et X sont définis comme dans la formule I.

6. Procédé selon une ou plusieurs des revendications 1 à 5, caractérisé en ce qu'on réalise le procédé en présence d'un solvant organique ou inorganique inerte dans les conditions de réaction ou d'un mélange de plusieurs solvants correspondants.

7. Procédé selon une ou plusieurs des revendications 1 à 6, caractérisé en ce le procédé est réalisé en présence d'un solvant organique inerte.

8. Procédé selon une ou plusieurs des revendications 1 à 7, caractérisé en ce que l'on fait réagir le composé de formule II en quantité équimolaire ou en léger excès par rapport au composé de formule III.

9. Procédé selon la revendication 8, caractérisé en ce que le rapport molaire II:III est d'environ 1:1 à 1,2:1.

10. Procédé selon une ou plusieurs des revendications 1 à 9, caractérisé en ce que les températures de réaction sont d'environ 0°C à 140°C.

11. Procédé de préparation de composés de formule II, 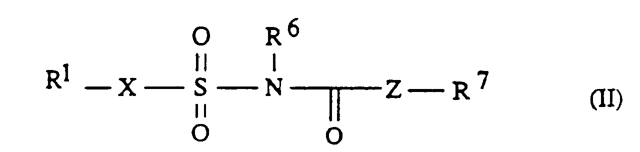 dans laquelle R¹, R⁶, R⁷, X et Z sont définis comme dans la formule II selon une ou plusieurs des revendications 1 à 4 ou 1 à 3 et 5, caractérisé en ce que, l'on fait réagir des sulfonamides de formule VI avec des chlorures d'acides de formule VII, 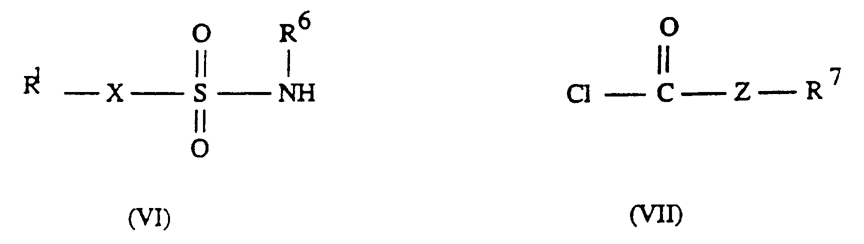 dans laquelle R¹, R⁶, R⁷, X et Z sont définis comme dans la formule II.

12. Utilisation de composés de formule II, tels qu'ils sont définis dans la revendication 11, pour la préparation de sulfonylurées.
